# EUROPÄISCHE PATENTSCHRIFT

(11) **EP 2 272 348 B1**
(45) Veröffentlichungstag und Bekanntmachung des Hinweises auf die Patenterteilung: **04.03.2015**
(21) Anmeldenummer: 09164779.2
(22) Anmeldetag: 07.07.2009
(51) Int. Cl.: A01N 43/80, A01N 59/20, A01P 1/00

(54) **Biozide Mittel**
Biocidal agent
Moyen biocide

(43) Veröffentlichungstag der Anmeldung: 12.01.2011
(73) Patentinhaber: LANXESS Deutschland GmbH, 50569 Köln (DE)
(72) Erfinder: Uhr, Hermann, Dr., 51373 Leverkusen (DE)

(56) Entgegenhaltungen:
- EP-A- 0 910 952
- EP-A- 0 913 090
- EP-A- 0 983 723
- EP-A- 1 005 271
- EP-A- 1 120 040
- JP-A- 2 304 005

## Beschreibung

Die vorliegende Erfindung betrifft lagerstabile biozide Mittel enthaltend als Isothiazolinone nur 2-Methyl-2*H*-isothiazol-3-on (MIT) und 1,2-Benzisothiazolin-3-on (BIT) und/oder dessen Salze sowie stabilisierende Mengen an Kupfer(II)-Ionen, ein Verfahren zur Konservierung von technischen Materialien mittels vorgenannter biozider Mittel, sowie die damit behandelten technischen Materialien.

In der Literatur sind vielfältige Methoden beschrieben, Isothiazolinone vor einer chemischen Zersetzung zu schützen. Hierbei handelt es sich im Wesentlichen um die Stabilisierung von 3:1 Mischungen aus 5-Chlor-2-methyl-4-isothiazolin-3-on (CMIT) und MIT, wie sie bei der technischen Herstellung typischerweise anfallen.

Das zur Zersetzung neigende CMIT muss stabilisiert werden, während MIT unter anwendungs- oder lagertypischen Bedingungen stabil ist.

EP 0 721 736 A beschreibt stabilisierte Isothiazolinonlösungen von CMIT und MIT in denen die Stabilierung durch nicht chelatisierte Kupfer (II)-Ionen erreicht wird, wobei das Gewichtsverhältnis von Kupfer (II)-Ionen und Isothiazolinonen zwischen 0,0008 : 1,5 und 0,02 : 1,5 liegt.

Des weiteren beschreibt die EP 0 749 689 A die Verhinderung von Ausfällungen in Isothiazolinonformulierungen durch den Einsatz von Kupfer (II)-Ionen und Metallnitraten. Dabei werden zur Stabilisierung 0,1 bis 25 % Metallnitrate wie beispielsweise Magnesiumnitrat und ca. 0,1 bis 100 ppm Kupfer (II)-Ionen eingesetzt.

Aus EP 1 369 461 A ist bereits bekannt, dass man den Abbau von Isothiazolinonen in wässrigen Farbsystemen dadurch verlangsamen kann, dass man 1 bis 200 ppm Kupfer (II)-Ionen zur Stabilisierung einsetzt. Im Verhältnis zum eingesetzten Isothiazolinon sind hier die Kupfermengen vergleichsweise hoch.

In der EP 0 408 215 werden nicht in das Holz penetrierende, Isothiazolinon-haltige Farben beschrieben, die durch nicht wasserlösliche Kupferverbindungen, wie beispielsweise Kupfersalze von Fettsäuren stabilisiert sind.

Weiterhin ist aus EP 1 044 609 A, EP 0 910 952 und EP 0 913 090 A bekannt, Kupfersalze als Co-Stabilisatoren neben hohen Mengen starker Oxidationsmittel wie beispielsweise Iodaten, Periodaten, Chloraten, Perchloraten und organische Oxidationsmittel einzusetzen.

Aufgrund der vorstehend beschriebenen Stabilität von MIT und auch BIT ist eine Stabilisierung von bioziden Mitteln enthaltend MIT oder BIT einzeln oder in Kombination typischerweise nicht erforderlich (siehe auch EP 1 005 271 A).

Allerdings haben jüngere Untersuchungen gezeigt, dass es bei bioziden Mitteln enthaltend halogenfreie Isothiazolinone wie beispielsweise bioziden Mitteln enthaltend BIT und MIT bei einer Lagerung bei höheren Temperaturen, wie sie beispielsweise in subtropischen oder tropischen Klimaten häufig anzutreffen sind, zu deutlichen Verfärbungen, Abbau der Wirkstoffe und Ausfällungen kommen kann.

Es bestand daher die Aufgabe diese Nachteile zu vermeiden.

Es wurden nun biozide Mittel gefunden, die enthalten
- als Isothiazolinone nur 2-Methyl-2H-isothiazol-3-on und 1,2-Benzisothiazolin-3-on und/oder dessen Salze und
- 1 bis 500, bevorzugt 5 bis 500, besonders bevorzugt 10 bis 300 Gewichts-ppm Kupfer(II)-Ionen bezogen auf den gesamten Gewichtsanteil des oder der Isothiazolinone im bioziden Mittel.

Die bioziden Mittel enthalten als Isothiazolinone nur 2-Methyl-2*H*-isothiazol-3-on und 1,2-Benzisothiazolin-3-on und/oder dessen Salze.

Die erfindungsgemäßen bioziden Mittel sind vorzugsweise flüssig. Der Begriff "flüssiges biozides Mittel" bedeutet im Rahmen der Erfindung, dass das Mittel bei Raumtemperatur im flüssigen Aggregatzustand vorliegt und der Gehalt an festen Bestandteilen 0 bis 1 Gew.-%, bevorzugt 0 bis 0,5 Gew.-% beträgt. Besonders bevorzugt sind die flüssigen bioziden Mittel frei von festen Bestandteilen.

Die flüssigen bioziden Mittel können wässrig oder nichtwässrig sein, wobei unter nichtwässrigen bioziden Mitteln im Sinne der Erfindung solche verstanden werden, die bezogen auf das Gesamtgewicht der bioziden Mittel einen Anteil von weniger als 5 Gew.-%, bevorzugt einen Anteil von weniger als 2 Gew.-% Wasser aufweisen.

Der Gewichtsanteil an Isothiazolinonen in den bioziden Mitteln kann beispielsweise 0,1 bis, wegen des erforderlichen Kupferanteils gerundet, 100 % betragen. Insbesondere bei Verwendung von 2-n-Octyl-4-isothiazolin-3-on, das bei Raumtemperatur flüssig ist, lassen sich sehr hohe bis maximale Anteile an Isothiazolinonen auch in Form von flüssigen bioziden Mitteln verwirklichen.

Im Falle wässriger, flüssiger biozider Mittel ist ein Gewichtsanteil an Isothiazolinonen in den bioziden Mitteln von 0,1 bis 50 Gew.-% bevorzugt, ein Gewichtsanteil von 3 bis 20 Gew.-% besonders bevorzugt, ein Anteil von 5 bis 15 Gew.-% ganz besonders bevorzugt.

Im Rahmen der Erfindung sind flüssige, wässrige biozide Mittel ganz besonders bevorzugt.

In einer Ausführungsform können die bioziden Mittel weiterhin zumindest ein Oxidationsmittel enthalten, das ausgewählt ist aus der Gruppe: Iodat, Periodat, Perchlorat, Chlorat, Bromat, organische Oxidationsmittel, wobei Iodat, Periodat und Bromat bevorzugt und Iodat besonders bevorzugt ist. Vorgenannte anorganischen Oxidationsmittel können in die bioziden Mittel in an sich bekannter Weise beispielsweise in Form ihrer Alkalimetallsalze eingebracht werden.

Der Gehalt der bioziden Mittel an zumindest einem Oxidationsmittel kann beispielsweise 50 bis 10.000, bevorzugt 100 bis 1.000, besonders bevorzugt 10 bis 300 Gewichts-ppm bezogen auf ihren Gewichtsgehalt an Isothiazolinonen insgesamt betragen.

In einer bevorzugten Ausführungsform sind die bioziden Mittel frei von oben genannten Oxidationsmitteln, was im Sinne der Erfindung einen Gehalt von weniger als 2 Gewichts-ppm, bevorzugt völlige Abwesenheit der Oxidationsmittel bezogen auf die bioziden Mittel bedeutet.

Bevorzugte, flüssige, wässrige biozide Mittel sind solche die im Wesentlichen frei von organischen Lösungsmitteln sind. Im Wesentlichen frei von organischen Lösungsmitteln bedeutet im Rahmen der Erfindung einen Gewichtsanteil an organischen Lösungsmittel in den bioziden Mitteln von 0 bis 3 Gew.-%, vorzugsweise 0 bis 1 Gew.-%, bevorzugt völlige Abwesenheit von organischen Lösungsmitteln, wobei die Isothiazolinone nicht als organisches Lösungsmittel gelten, selbst wenn sie flüssig sind.

In einer bevorzugten Ausführungsform weisen die bioziden Mittel einen Nitratgehalt von 1.000 Gewichts-ppm oder weniger, bevorzugt von 250 Gewichts-ppm oder weniger, besonders bevorzugt von 50 Gewichts-ppm oder weniger bezogen auf das Gesamtgewicht der bioziden Mittel auf. In einer anderen Ausführungsform beträgt der Nitratgehalt der bioziden Mittel weniger als 20 Gewichts-ppm.

Bevorzugte flüssige, wässrige biozide Mittel sind solche, die zwei Isothiazolinone enthalten, wobei die Isothiazolinone 2-Methyl-2H-isothiazol-3-on (MIT) und 1,2-Benzisothiazolin-3-on (BIT) oder dessen Salze sind und die weiterhin 1 bis 500, bevorzugt 5 bis 500, besonders bevorzugt 10 bis 300 Gewichts-ppm Kupfer(II)-Ionen bezogen auf den Gewichtsanteil der vorgenannten Isothiazolinone im bioziden Mittel enthalten.

Diese bevorzugten flüssigen, wässrigen bioziden Mittel enthalten vorzugsweise 0,5 bis 20 Gew.-% BIT oder dessen Salze jeweils berechnet auf freies BIT, bevorzugt 1 bis 15 Gew.-% und 0,5 bis 20 Gew.-% MIT, bevorzugt 1 bis 15 Gew.-% MIT.

Das Gewichtsverhältnis von BIT zu MIT kann in einem breiten Bereich variieren, bevorzugt beträgt das Gewichtsverhältnis von BIT zu MIT 1:10 bis 10:1, bevorzugt 1:5 bis 5:1.

Vorzugsweise sind vorgenannte flüssige, wässrige biozide Mittel im Wesentlichen frei von organischen Lösungsmitteln.

Vorzugsweise wird BIT in Form seiner Alkalimetallsalze, beispielsweise in Form des Lithium-, Natrium- oder Kaliumsalzes eingesetzt. Die Herstellung der Alkalimetallsalze von BIT erfolgt üblicherweise durch Umsetzung von BIT mit dem entsprechenden Alkalimetallhydroxid, wobei typischerweise 0,7 bis 1,2 Moläquivalente des Alkalimetallhydroxids bezogen auf BIT, bevorzugt 0,8 bis 1,1 Molequivalente eingesetzt werden.

Die bevorzugten flüssigen, wässrigen bioziden Mittel weisen bei Verwendung von BIT-Salzen dann typischerweise einen pH-Wert von 7 bis 11 unter Standardbedingungen auf, vorzugsweise einen pH-Wert von 8 bis 10.

Die erfindungsgemäßen bioziden Mittel eignen sich insbesondere zur Konservierung von technischen Materialien, die anfällig sind gegen den Befall durch Mikroorganismen.

Die Erfindung betrifft daher weiterhin die Verwendung der erfindungsgemäßen bioziden Mittel zum Schutz von technischen Materialien vor Befall durch und der Bekämpfung von Mikroorganismen, sowie ein Verfahren zum Schutz von technischen Materialien vor Befall und/oder Zerstörung durch Mikroorganismen, das dadurch gekennzeichnet ist, dass man die erfindungsgemäßen bioziden Mittel auf den Mikroorganismus oder dessen Lebensraum einwirken lässt. Die Einwirkung kann dabei in verdünnter oder unverdünnter Form erfolgen.

Die Erfindung betrifft außerdem technische Materialien, erhältlich durch Behandlung von technischen Materialien mit den erfindungsgemäßen bioziden Mitteln, sowie technische Materialien enthaltend
- als Isothiazolinone nur 2-Methyl-2*H*-isothiazol-3-on und 1,2-Benzisothiazolin-3-on und/oder dessen Salze und
- 1 bis 500, bevorzugt 5 bis 500, besonders bevorzugt 10 bis 300 Gewichts-ppm Kupfer(II)-Ionen bezogen auf den gesamten Gewichtsanteil des oder der Isothiazolinone im technischen Material.

Bevorzugte technische Materialien sind funktionelle Flüssigkeiten und wasserhaltige technische Produkte, wie beispielsweise:
- Anstrichmittel, Farben, Putze und sonstige Beschichtungsmittel
- Stärkelösungen und -slurrys oder andere auf Basis von Stärke hergestellte Produkte wie beispielsweise Druckverdicker
- Slurrys anderer Rohstoffe wie beispielsweise Farbpigmente, wie beispielsweise Eisenoxidpigmente, Russpigmente, Titandioxidpigmente, oder Slurrys von anorganischen Füllstoffen und Pigmenten wie Kaolin, Calciumcarbonat, Gips, Bentonit, Magnesiumsilicate, Smectid oder Talkum.
- Bauchemische Produkte wie beispielsweise Betonadditive beispielsweise auf Basis von Melasse, Ligninsulfonat oder Polyacrylaten, Bitumenemulsionen oder Fugendichtungsmassen;
- Leime oder Klebstoffe auf Basis bekannter tierischer, pflanzlicher oder synthetischer Rohstoffe;
- Polymerdispersionen auf Basis von beispielsweise Polyacrylaten, Polystyrolacrylaten, Styrolbutadien, Polyvinylacetaten;
- Detergentien und Reinigungsmittel für Industrie und Haushalt
- Mineralöle und Mineralölprodukte wie beispielsweise Dieselkraftstoffe)
- Kühlschmierstoffe zur Metallverarbeitung beispielsweise auf Basis von Mineralöl-haltigen, halbsynthetischen oder synthetischen Konzentraten
- Hilfsmittel für die Leder-, Textil- oder photochemische Industrie
- Vor- und Zwischenprodukte der chemischen Industrie, beispielsweise bei der Farbstoffproduktion und -lagerung
- Tinten oder Tuschen
- Wachsen und Tonemulsionen

Mikroorganismen im Sinne der Erfindung sind beispielsweise Bakterien, Schimmelpilze, Hefen und Schleimorganismen. Beispielhaft, jedoch ohne Beschränkung darauf seien die folgenden Mikroorganismen genannt:
Alternaria wie Alternaria tenuis, Aspergillus wie Aspergillus niger, Chaetomium wie Chaetomium globosum, Fusarium wie Fusarium solani, Lentinus wie Lentinus tigrinus, Penicillium wie Penicillium glaucum, Polyporus, wie Polyporus versicolor, Aureobasidium, wie Aureobasidium pullulans, Sclerophoma, wie Sclerophoma pityophila, Trichoderma, wie Trichoderma viride.
Alcaligenes wie Alcaligenes faecalis, Bacillus wie Bacillus subtilis, Escherichia wie Escherichia coli,
Pseudomonas wie Pseudomonas aeruginosa oder Pseudomonas fluorescens, Staphylococcus wie Staphylococcus aureus;
Candida wie Candida albicans, Geotrichum wie Geotrichum candidum, Rhodotorula wie Rhodotorula rubra.

Die erfindungsgemäßen bioziden Mittel können neben den Isothiazolinonen zumindest einen weiteren bioziden Wirkstoff enthalten, der kein Isothiazolinon ist, wobei die weiteren bioziden Wirkstoffe Algizide, Fungizide oder Bakterizide sein können.

Bevorzugte Algizide sind Triazinverbindungen, wie beispielsweise Terbutryn, Cybutryn, Propazin oder Terbuton; Harnstoffverbindungen, wie beispielsweise Diuron, Benzthiazuron, Isoproturon, Methabenzthiazuron und Tebuthiuron oder Uracile, wie beispielsweise Terbacil.

Bevorzugte Fungizide sind Azaconazol, Bromuconazol, Cyproconazol, Dichlobutrazol, Diniconazol, Hexaconazol, Metaconazol, Penconazol, Propiconazol, Tebuconazol, Methfuroxam, Carboxin, Fenpiclonil, Butenafin, Imazalil, Thiabendazol, 1-Hydroxy-2-pyridinthion sowie ihre Cu-, Na-, Fe-, Mn-, Zn-Salze; Tetrachlor-4-methylsulfonylpyridin, 3-Iod-2-propinyl-n-butylcarbamat, Bethoxazin, 2,4,5,6-Tetrachlorophthalodinitril, Triadimefon und Carbendazim.

Bevorzugte Bakterizide sind: Glutaraldehyd, Pyrithion und seine Salze, 2-Brom-2-nitro-1,3-propandiol, o-Phthaldialdehyd, 2,2-Dibrom-3-nitril-propionamid, 1,2-Dibrom-2,4-dicyanobutan, p-Hydroxybenzoesäure, Chlorophen, 3-Methyl-4-chlorphenol, o-Phenylphenol, p-tert.-Amylphenol, quaternäre Ammoniumsalze wie beispielsweise Benzalkoniumchlorid, Benzyldimethyltetradecylammoniumchlorid, Benzyldimethyldodecylammoniumchlorid, Dichlorbenzyldimethylalkylammoniumchlorid, Didecyldimethylammoniumchlorid, Dioctyldimethylammoniumchlorid, N-Hexadecyltrimethylammoniumchlorid, N-Cyclohexyldiazeniumdioxid Kaliumsalz, Formaldehyd oder Formadehyddepotstoffe wie beispielsweise N-(2-Hydroxypropyl)-amino-methanol, Benzylalkohol-(hemi)-formal, N-Methylolchloracetamid, 1,3-Bis(hydroxymethyl)-5,5-dimethylimidazolidine-2,4-dione (DMDMH), Tetramethylol-acetylen-diharnstoff (TMAD), Ethylenglycol-hemiformal, Ethylenglycol-bis-hemiformal, Hexahydro-s-triazin, 7-Ethylbicyclooxazolidine, 3,3'-Methylene-[5-methylbisoxazolidine], Dimethylolharnstoff, N-methylolharnstoff, Methylenbismorpholin, Natrium-N-(hydroxymethyl)glycinat.

Sofern die erfindungsgemäßen bioziden Mittel neben den Isothiazolinonen zumindest einen weiteren bioziden Wirkstoff enthalten, sind bei wässrigen bioziden Mitteln folgende Biozide bevorzugt:
Formaldehydabspalter wie beispielsweise Benzylhemiformal, Tetramethylol-acetylendiharnstoff (TMAD), 1,3-Bis(hydroxymethyl)-5,5-dimethylimidazolidine-2,4-dione (DMDMH), Dimethylolharnstoff, N-Methylolharnstoff, Ethylenglykolhemiformal, Ethylenglykol-bis-hemiformal, Phenole wie beispielsweise p-Chlor-m-kresol, p-tert.-Amylphenol, o-Phenyl-phenol, Chlorophen, Quarternäre Ammoniumsalze wie beispielsweise Benzalkoniumchlorid, Benzyldimethyltetradecylammoniumchlorid, Benzyldimethyldodecylammoniumchlorid, Dichlor-benzyldimethylalkylammoniumchlorid, Didecyldimethylammoniumchlorid, Dioctyl-dimethylammoniumchlorid, N-Hexadecyltrimethylammoniumchlorid sowie N-Cyclohexyldiazeniumdioxid Kaliumsalz und 2-Dibrom-2,4-dicyanobutan.

In einer besonders bevorzugten Ausführungsform der Erfindung enthalten die erfindungsgemäßen bioziden Mittel neben den Isothiazolinonen keine weiteren Biozide.

Die Anwendungskonzentrationen der erfindungsgemäßen bioziden Mittel richtet sich nach der Art und dem Vorkommen der zu bekämpfenden Mikroorganismen, der mikrobiellen Ausgangsbelastung sowie nach der Zusammensetzung des zu schützenden technischen Materials. Die optimale Einsatzmenge für eine bestimmte Anwendung kann vor dem Praxiseinsatz in an sich und dem Fachmann hinlänglich bekannter Weise durch Testreihen im Labor einfach ermittelt werden. Im allgemeinen liegen die Anwendungskonzentrationen im Bereich von 0,01 bis 5 Gew. %, vorzugsweise von 0,05 bis 1,0 Gew.-% der erfindungsgemäßen bioziden Mittel, bezogen auf das zu schützende Material.

Der besondere Vorteil der Erfindung liegt darin, dass bereits mit geringsten Mengen von Kupfer(II)-Ionen eine Langzeitstabilität der erfindungsgemäßen bioziden Mittel erreicht werden kann, und im Vergleich zu unstabilisierten bioziden Mitteln Wirkstoffabbau, Verfärbung und Ausfallen von Zersetzungsprodukten effektiv unterbunden, zumindest jedoch deutlich verringert werden kann.

### Beispiele

### Beispiel 1

### Lösung A (unstabilisiert)

569,8 g demineralisiertes Wasser wurden vorgelegt, mit 17,86 g einer wässrigen Natriumhydroxid-Lösung (50 Gewichts-%), und 21,6 g Natriumchlorid versetzt und solange gerührt, bis alles gelöst war. Hierzu gab man 41,6 g BIT (86,5 Gew.-%, Hersteller I), das mit Wasser staubfrei gemacht wurde und rührte solange bei Raumtemperatur, bis sich alles gelöst hatte. In diese Lösung gab man anschließend unter Rühren 69,1 g einer wässrigen MIT Lösung (52,10 Gew.-%). Man erhielt eine klare, nahezu farblose Lösung mit einem pH-Wert von 8,6.

### Lösung B (stabilisiert)

92,86 g der oben beschriebenen Lösung A wurden vorgelegt und mit 7,14 g einer 0,001 molaren Lösung von Kupfer(II)nitrat unter Rühren versetzt. Man erhielt eine klare, farblose Lösung mit einem pH-Wert von 8,6.

Beide Lösungen wurden jeweils bei 20 °C und 65 °C gelagert und jeweils nach 7 Tagen das Aussehen bewertet. Die Ergebnisse sind in Tabelle 1 angegeben.

**Tabelle 1:**

| | Lösung A (unstabilisiert) | | Lösung B (stabilisiert) | |
|---|---|---|---|---|
| | 7 Tage 20 °C | 7 Tage 65 °C | 7 Tage 20 °C | 7 Tage 65 °C |
| Aussehen | Farblose, klare Lösung | Fast schwarze Lösung mit gelbem Bodensatz | Farblose, klare Lösung | Farblose, klare Lösung |

### Beispiel 2

### Lösung C (unstabilisiert)

569,5 g demineralisiertes Wasser wurden vorgelegt, mit 17,86 g wässrigen NatriumhydroxidLösung (50 Gewichts-%), und 21,6 g Natriumchlorid versetzt und solange gerührt, bis alles gelöst war. Hierzu gab man 42,0 g BIT (85,74 Gew.-%, Hersteller II), das mit Wasser staubfrei gemacht wurde und rührte solange bei Raumtemperatur, bis sich alles gelöst hatte. In diese Lösung gab man anschließend unter Rühren 69,1 g einer wässrigen MIT Lösung (52,10 Gew.-%). Man erhielt eine klare, nahezu farblose Lösung mit einem pH-Wert von 8,8.

### Lösung D (stabilisiert)

92,86 g der oben beschriebenen Lösung A wurden vorgelegt und mit 7,14 g einer 0,001 molaren Lösung von Kupfer(II)nitrat unter Rühren versetzt.

Man erhielt eine klare, farblose Lösung mit einem pH-Wert von 8,8.

Beide Lösungen wurden jeweils bei 20 °C und 65 °C gelagert und nach jeweils 7 Tagen das Aussehen bewertet. Die Ergebnisse sind in Tabelle 2 angegeben.

**Tabelle 2:**

| | Lösung C (unstabilisiert) | | Lösung D (stabilisiert) | |
|---|---|---|---|---|
| | 7 Tage 20 °C | 7 Tage 65 °C | 7 Tage 20 °C | 7 Tage 65 °C |
| Gehalt BIT (Gew.-%) | 4,6% | 3,3 % | 4,3 % | 4,2% |
| Gehalt MIT (Gew.-%) | 4,8 % | 2,8 % | 4,5 % | 4,4 % |
| Aussehen | Farblose, klare Lösung | Dunkelbraune Lösung mit gelben Bodensatz | Farblose, klare Lösung | Farblose, klare Lösung |

## Patentansprüche

1. Biozide Mittel enthaltend
• als Isothiazolinone nur 2-Methyl-2*H*-isothiazol-3-on und 1,2-Benzisothiazolin-3-on und/oder dessen Salze und
• 1 bis 500 Gewichts-ppm Kupfer(II)-Ionen bezogen auf den gesamten Gewichtsanteil des oder der Isothiazolinone im bioziden Mittel.

2. Biozide Mittel nach Anspruch 1, **dadurch gekennzeichnet, dass** sie flüssig sind.

3. Biozide Mittel nach Anspruch 2, **dadurch gekennzeichnet, dass** sie einen Gewichtsanteil an organischen Lösungsmitteln von 0 bis 3 Gew.-% aufweisen.

4. Biozide Mittel nach einem der Ansprüche 1 bis 3, **dadurch gekennzeichnet, dass** sie weiterhin zumindest ein Oxidationsmittel enthalten, das ausgewählt ist aus der Gruppe: Iodat, Periodat, Perchlorat, Chlorat, Bromat und organische Oxidationsmittel.

5. Biozide Mittel nach einem der Ansprüche 1 bis 3, **dadurch gekennzeichnet, dass** sie frei von Iodat, Periodat, Perchlorat, Chlorat, Bromat und organischen Oxidationsmitteln sind.

6. Biozide Mittel nach einem der Ansprüche 1 bis 5, **dadurch gekennzeichnet, dass** sie einen Nitratgehalt von 1.000 Gewichts-ppm oder weniger bezogen auf das Gesamtgewicht der bioziden Mittel aufweisen.

7. Verwendung von bioziden Mitteln nach einem der Ansprüche 1 bis 6 zum Schutz von technischen Materialien vor Befall durch und der nicht therapeutischen Bekämpfung von Mikroorganismen.

8. Verfahren zum Schutz von technischen Materialien vor Befall und/oder Zerstörung durch Mikroorganismen, **dadurch gekennzeichnet, dass** man biozide Mittel nach einem der Ansprüche 1 bis 6 auf den Mikroorganismus oder dessen Lebensraum einwirken lässt.

9. Technische Materialien, erhältlich durch Behandlung von technischen Materialien mit den bioziden Mitteln nach einem der Ansprüche 1 bis 6.

## Claims

1. Biocidal compositions comprising
• as isothiazolinones, only 2-methyl-2H-isothiazol-3-one and 1,2-benzisothiazolin-3-one and/or salts thereof and
• 1 to 500 ppm by weight of copper (II) ions, based on the total weight fraction of the isothiazolinone or isothiazolinones in the biocidal composition.

2. Biocidal compositions according to Claim 1, **characterized in that** they are liquid.

3. Biocidal compositions according to Claim 2, **characterized in that** they have a weight fraction of organic solvents of from 0 to 3% by weight.

4. Biocidal compositions according to one of Claims 1 to 3, **characterized in that** they further comprise at least one oxidizing agent which is selected from the group: iodate, periodate, perchlorate, chlorate, bromate and organic oxidizing agents.

5. Biocidal compositions according to one of Claims 1 to 4, **characterized in that** they are free from iodate, periodate, perchlorate, chlorate, bromate and organic oxidizing agents.

6. Biocidal compositions according to one of Claims 1 to 5, **characterized in that** they have a nitrate content of 1000 ppm by weight or less, based on the total weight of the biocidal compositions.

7. Use of biocidal compositions according to one of Claims 1 to 6 for protecting technical materials against infestation by and the nontherapeutic control of microorganisms.

8. Method for protecting technical materials against infestation and/or destruction by microorganisms, **characterized in that** biocidal compositions according to one of Claims 1 to 6 are allowed to act on the microorganism or its habitat.

9. Technical materials obtainable by treating technical materials with the biocidal compositions according to one of Claims 1 to 6.

## Revendications

1. Agents biocides contenant .
- en tant qu'isothiazolinones, seulement de la 2-méthyl-2H-isothiazol-3-one et de la 1,2-benzisothiazolin-3-one et/ou leurs sels, et
- 1 à 500 ppm en poids d'ions cuivre (II) par rapport à la proportion en poids totale de la ou des isothiazolinones dans l'agent biocide.

2. Agents biocides selon la revendication 1, **caractérisés en ce qu'**ils sont liquides.

3. Agents biocides selon la revendication 2, **caractérisés en ce qu'**ils présentent une proportion en poids de solvants organiques de 0 à 3 % en poids.

4. Agents biocides selon l'une quelconque des revendications 1 à 3, **caractérisés en ce qu'**ils contiennent en outre au moins un oxydant, qui est choisi dans le groupe constitué par : l'iodate, le periodate, le perchlorate, le chlorate, le bromate et les oxydants organiques.

5. Agents biocides selon l'une quelconque des revendications 1 à 3, **caractérisés en ce qu'**ils sont exempts d'iodate, de periodate, de perchlorate, de chlorate, de bromate et d'oxydants organiques.

6. Agents biocides selon l'une quelconque des revendications 1 à 5, **caractérisés en ce qu'**ils présentent une teneur en nitrate de 1 000 ppm en poids ou moins par rapport au poids total des agents biocides.

7. Utilisation d'agents biocides selon l'une quelconque des revendications 1 à 6 pour la protection de matériaux techniques contre l'attaque par des microorganismes et la lutte non thérapeutique contre les microorganismes.

8. Procédé de protection de matériaux techniques contre l'attaque et/ou la destruction par des microorganismes, **caractérisé en ce que** des agents biocides selon l'une quelconque des revendications 1 à 6 sont laissés agir sur les microorganismes ou leur habitat.

9. Matériaux techniques, pouvant être obtenus par traitement de matériaux techniques avec les agents biocides selon l'une quelconque des revendications 1 à 6.
